# EUROPEAN PATENT APPLICATION

(11) **EP 1 743 659 A1**
(43) Date of publication of application: **17.01.2007**
(21) Application number: 06012672.9
(22) Date of filing: 20.06.2006
(51) Int. Cl.: A61L 17/04

(54) **Monofilament sutures made from a composition containing ultra high molecular weight polyethylene**

(30) Priority: 13.07.2005 US 698671 P
(71) Applicant: Tyco Healthcare Group LP, Norwalk, Connecticut 06856 (US)
(72) Inventor: Roby, Mark S., Killingworth CT 06419 (US)
(74) Representative: HOFFMANN EITLE

(57) **Abstract**

The present disclosure provides surgical sutures made from a composition including an ultra high molecular weight polyethylene. Methods for producing such sutures and uses thereof are also provided.

## Description

### CROSS-REFERENCE TO RELATED APPLICATIONS

This application claims the benefit of U.S. Provisional Patent Application No. 60/698,671 filed July 13, 2005, the entire disclosure of which is hereby incorporated by reference herein.

### BACKGROUND

### 1. Technical Field

The present disclosure relates to surgical sutures, and particularly to monofilament sutures made from a composition containing ultra high molecular weight polyethylene.

### 2. Background of the Related Art

Polyolefin sutures are known in the art. Such sutures are non-absorbable and generally include polypropylene or polymeric combinations of ethylene and propylene. The polymeric components of the polyolefin sutures are generally melt spun to produce filaments for use in fabricating the surgical suture strands. Polypropylene sutures are advantageously produced as monofilament sutures.

Various methods are known for making polypropylene sutures. For example, U.S. Patent No. 5,217,485 to Liu et al. discloses a process for making a polypropylene monofilament suture by melt extruding the monofilament, stretching the solidified monofilament, then allowing the monofilament to equilibrate, or "rest", prior to annealing.

While currently available polypropylene monofilament sutures provide satisfactory performance, there remains room for improvements to be made in the area of non-absorbable monofilament sutures.

### SUMMARY

It has now been found that monofilament sutures can be prepared from polymeric compositions containing ultra high molecular weight polyethylene. In embodiments, the ultra high molecular weight polyethylene may possess a weight average molecular weight of greater than about 400,000. A method for fabricating an ultra high molecular weight polyethylene suture is also provided herein.

The present disclosure also provides a surgical needle-suture combination including a surgical needle and a suture attached to the needle, the suture including a monofilament of an ultra high molecular weight polyethylene possessing a weight average molecular weight of greater than about 400,000.

Methods of suturing wounds with sutures of the present disclosure are also provided. Such methods include providing a monofilament suture having an ultra high molecular weight polyethylene possessing a weight average molecular weight of greater than about 400,000 attached to a needle to form a needled suture, and passing the needled suture through tissue to create wound closure.

### BRIEF DESCRIPTION OF THE DRAWING(S)

FIG. 1 is a schematic illustration of apparatus which is suitable for carrying out the suture manufacturing process described herein; and
FIG. 2 is a depiction of a needled suture in accordance with the present disclosure.

### DETAILED DESCRIPTION OF PREFERRED EMBODIMENT(S)

All composition percentages listed herein shall be understood to be by weight unless otherwise indicated. All quantities set forth below, except in the claims, shall be understood to be modified by the term "about".

The present disclosure relates to a composition from which monofilament sutures can be produced, such as, for example, by melt extrusion, or "spinning", of polymeric compositions containing ultra high molecular weight polyethylene. Typical compositions include ultra high molecular weight polyethylene. The ultra high molecular weight polyethylene can be used alone or blended with other polymers such as, for example, polyvinyl ether, poly(phenylene ether), acetal resin, thermoplastic polyester, polycarbonate, cellulose ester, thermoplastic polyamide, polyetheramide, polyesteramide, thermoplastic polyurethane, polyvinylhalide, polyvinylidene halide, halogenated polyolefin, acrylic resin, polystyrene, polyacrylonitrile, polyvinylester, polyimide, polyetherimide, polysulfone, and combinations thereof.

In embodiments, the ultra high molecular weight polyethylene may be blended with one or more materials such as polyethylenes of lower molecular weight, polypropylene, or copolymers of polyethylene and polypropylene. Blends of the ultra high molecular weight polyethylene with one or more other thermoplastic resins may contain from about 5 to about 95 weight percent, in embodiments from about 20 to about 80 weight percent, ultra high molecular weight polyethylene, the balance of the blend containing a different thermoplastic resin, such as, for example, a lower molecular weight polyethylene, isotactic polypropylene, atactic polypropylene, or syndiotactic polypropylene, and the like.

Alternatively, the composition containing ultra high molecular weight polyethylene can include a copolymer made from ultra high molecular weight polyethylene. Specifically, random, block, or graft copolymers can be formed from the copolymerization of ultra high molecular weight polyethylene with one or more monomers or sequences of monomers copolymerizable therewith including, but not limited to, other alpha-olefins such as, for example, polypropylene units, e.g., isotactic, syndiotactic and/or atactic polypropylenes.

Useful ultra high molecular weight polyethylene resins include those possessing a weight average molecular weight (Mw) of greater than about 400,000, typically greater than about 1,000,000. Useful ultra high molecular weight polyethylene resins may advantageously possess a melting point of from about 144° C to about 152° C. Ultra high molecular weight polyethylene resins which can be used herein include those sold under the trade names DYNEEMA and SPECTRA which are available from DSM (Heerlen, the Netherlands).

The composition utilized to form a suture of the present disclosure may optionally include a fatty acid diester to reduce fraying and facilitate suture formation. The fatty acid diester may include a diester of a polyalkylene glycol. Suitable fatty acids include C₁₀-C₂₆ fatty acids such as stearic, lauric, palmitic, myristic, arachidic, behenic, and similar acids. Suitable polyalkylene glycols include C₂-C₆ alklyene glycols, such as polyethylene glycols and polypropylene glycols.

It is contemplated that it may be desirable to dye the present sutures in order to increase visibility of the suture in the surgical field. Dyes known to be suitable for incorporation in sutures can be used. Such dyes include, but are not limited to, carbon black, bone black, D&C Green No. 6, and D&C Violet No. 2 as described in the handbook of U.S. Colorants for Food, Drugs and Cosmetics by Daniel M. Marrion (1979). Typically, sutures may be dyed by adding up to about a few percent, in embodiments about 0.2%, of a dye to the resin prior to formation of the monofilament.

Methods for extruding and processing filaments of polyolefins are within the purview of those skilled in the art and can be used for forming monofilaments from a composition containing ultra high molecular weight polyethylene in accordance with this disclosure.

For example, one exemplary method for manufacture of stretched filaments from a composition containing ultra high molecular weight polyethylene includes subjecting a solution of the composition in a solvent to spinning or film-forming, followed by cooling to give a gel-like filament containing the solvent, and stretching the gel-like filament at a high stretch ratio. Such processes are described, for example, in European Patent No. 77590, and U.S. Patent Nos. 4,344,908, 4,413,110, 4,422,993, 4,430,383, and 5,202,073, the entire disclosures of each of which are incorporated herein by this reference.

In a first step of another exemplary method for making a suture filament, the composition containing ultra high molecular weight polyethylene may be heated to form a polymer melt. This melt may then be extruded and cooled to form a filament which can then be sent to further processing such as stretching. The melt may contain substantially no water or organic solvents, and no substances which would be incompatible with body tissue. The composition containing ultra high molecular weight polyethylene may contain some colorant to facilitate visualizing the suture filament during a surgical procedure.

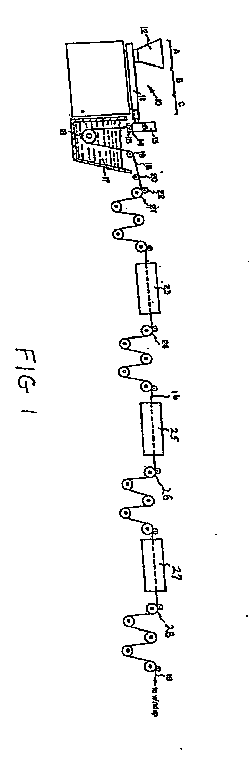

An exemplary process for manufacturing a suture is shown in FIG. 1, which schematically illustrates the extrusion and stretching operations of the monofilament manufacturing operation herein. Extruder unit 10 is of a known or conventional type and is equipped with controls for regulating the temperature of barrel 11 in various zones thereof, e.g., progressively higher temperatures in three consecutive zones A, B and C along the length of the barrel. Pellets or powder of the composition containing ultra high molecular weight polyethylene are introduced to the extruder through drier-hopper 12.

Motor-driven metering pump 13 delivers extruded resin at a constant rate to spin pack 14 and thereafter through spinneret 15 possessing one or more orifices of desired diameter to provide a molten monofilament 16 which then enters quench bath 17, e.g., containing water, where the monofilament solidifies. The distance monofilament 16 travels after emerging from spinneret 15 to the point where it enters quench bath 17, i.e., the air gap, can vary and can advantageously be from about 0.5 cm to about 100 cm, in embodiments from about 1 cm to about 20 cm. If desired, a chimney (not shown), or shield, can be provided to isolate monofilament 16 from contact by air currents which might otherwise affect the cooling of the monofilament in some unpredictable manner. In general, barrel zone A of the extruder can be maintained at a temperature of from about 180° C to about 230° C, zone B at from about 190° C to about 230° C, and zone C at from about 190° C to about 230° C. Additional temperature parameters include: metering pump block 13 at from about 190° C to about 230° C, spin pack 14 at from about 190° C to about 230° C, spinneret 15 at from about 190° C to about 230° C, and quench bath 17 at from about 30° C to about 80° C.

Entering quench bath 17, monofilament 16 is passed by driven roller 18 over idler rollers 19 and 20 and thereafter is wrapped around a first godet 21 provided with nip roll 22 to prevent slippage which might otherwise result from the subsequent stretching operation. Monofilament 16 passing from godet 21 is stretched in order to effect its orientation and thereby increase its tensile strength. Techniques and conditions for drawing (i.e., stretching polyolefin monofilaments) are within the purview of those skilled in the art. In one embodiment, described in detail below, the monofilament undergoes two heated draw operations.

As seen in FIG. 1 monofilament 16 may be drawn through heating unit 23, which can be an oven chamber or a hot water trough, by means of second godet 24 which rotates at a higher speed than first godet 21, thereby stretching the monofilament from about 4 times to about 7 times its original length, in embodiments from about 6 times to about 7 times its original length, and in other embodiments from about 6.5 times to about 6.8 times its original length. Where heating unit 23 is an oven chamber, its temperature may be advantageously maintained at from about 90° C to about 180° C, and in embodiments from about 110° C to about 160° C.

Monofilament 16 may be drawn a second time by passing it through heating unit 25, which can be an even chamber or a hot water trough, by means of third godet 26. The second draw may achieve a draw ratio of from about 1.1 to about 1.5, in embodiments from about 1.3 to about 1.4. Where heating unit 25 is an oven chamber, the temperature may be advantageously maintained at from about 100° C to about 170° C, in embodiments from about 120° C to about 150° C.

The monofilament may optionally be subjected to conditions which allow relaxation or shrinkage of the monofilament. Techniques and conditions suitable for achieving relaxation are within the purview of those skilled in the art. An example of a suitable technique is shown schematically in FIG. 1, wherein the monofilament is then passed through a third heating unit 27, e.g., maintained at a temperature of from about 100° C to about 180° C, in embodiments from about 110° C to about 175° C, by means of a fourth godet 28 to heat-treat the monofilament prior to the equilibration and annealing operations. This third heat treatment results in on-line relaxation, or shrinkage, of the monofilament, e.g., for a recovery of from about 65 percent to about 96 percent, in embodiments from about 70 percent to about 76 percent, of the stretched length of the monofilament. In order to accommodate this on-line shrinkage in the monofilament, the fourth godet 28 is driven at a speed which is somewhat less than that of the third godet 26.

Following stretching and orientation and, optionally, relaxation, monofilament from godet 28 is taken up on a spool (not shown). In some embodiments, the spool may then be set aside for a period of time sufficient to permit the monofilament to achieve a condition of equilibration. While the period of equilibration may vary depending on the particular composition containing ultra high molecular weight polyethylene selected and/or the conditions under which the resin is extruded, cooled and oriented, in most cases storage of the monofilament following its orientation occurs for at least about 2 days, in embodiments at least about 3 days, and in other embodiments at least about 4 days. It may be advantageous to store the spooled monofilament at ambient temperature, e.g., from about 20° C to about 23° C, and a relative humidity of about 50%.

In carrying out the annealing operation, the desired length of equilibrated suture may be wound around a creel and the creel placed in a heating cabinet maintained at the desired temperature, e.g., about 150° C, as described in U.S. Patent No. 3,630,205. The sutures can be cut to a desired length and heat set at that desired length. As shown in U.S. Patent No. 3,630,205, the creel may be rotated within the heating cabinet in order to ensure uniform heating of the monofilament or the cabinet may be of the circulating hot air type in which case uniform heating of the monofilament may be achieved without the need to rotate the creel. Thereafter, the creel with its annealed suture may be removed from the heating cabinet and, when returned to room temperature, the suture may be conveniently removed from the creel by cutting the wound monofilament at opposite ends of the creel. The annealed sutures, optionally attached to surgical needles, are than ready to be packaged and sterilized.

In an alternative embodiment, the monofilament suture may be drawn in two separate drawing steps in an amount from about 4 times to about 8.5 times and aged for less than about two days followed by annealing the filament to provide a suture. A more detailed disclosure of a suitable process of this type can be found in U.S. Patent No. 5,871,502.

In yet another alternative monofilament suture manufacturing process, the solidified monofilament may be allowed to dwell at ambient conditions for a predetermined period of time ranging from about 2 minutes to about 30 minutes prior to being drawn. A more detailed disclosure of a suitable process of this type can be found in U.S. Patent No. 5,456,696, the entire disclosure of which is incorporated by reference herein.

In yet another embodiment, drawn and oriented thermoplastic suture monofilaments may be subjected to a heat treatment to reduce modulus and otherwise improve physical properties by passing the suture filament through a radiant heater at a temperature maintained above the melting temperature of the suture monofilament. Operating conditions may be controlled so that the suture monofilament is subjected to a sufficient time/temperature exposure to modify the near-surface crystalline structure of the suture monofilament. The suture monofilament may be maintained under tension and typically drawn slightly during the heat treatment. Draw ratios of about 10 percent to about 20 percent or higher are possible with most materials. Treatment temperature may be from about 5° C to about 100° C or more above the melting temperature of the suture monofilament, with exposure time adjusted to obtain the desired effect on crystalline structure without penetrating too deeply within the suture monofilament.

Following the heat treatment, the monofilament may be relaxed and annealed to further increase crystallinity and decrease the degree of amorphous orientation, and then sterilized to render the monofilament suitable for use as a surgical suture. In a further variation of this embodiment, melt extruded, liquid quenched suture monofilaments may be drawn through a radiant heater maintained at a temperature above the melting temperature of the suture monofilaments. Draw speed, draw ratio, heater temperature and dwell time may be regulated to obtain the maximum stable draw ratio for the particular suture monofilament material. This draw ratio will generally be from about 3 times to about 6 times and from about 10 percent to about 30 percent more than the maximum stable draw ratio obtainable in the absence of the radiant heater. The use of the radiant heater to increase the overall stretch imparted to the suture filament during the initial drawing and orientation step thus further improves the ultimate physical properties obtainable for the suture.

The present monofilaments can optionally be treated to provide a roughened surface that have varied morphologies that exhibit non-uniform pitting and porosity. These characteristics assist with the ability of the monofilament to hold a knot and assist with the adhesion of materials to the surfaces of the monofilament. Plasma etching is one suitable method for producing irregularly roughened surfaces on a suture. The use of appropriate plasma gases and plasma operating conditions during etching may produce a monofilament suture having a surface with distinctive morphologies. The surface treatment process may be based on specific sequences of procedures that utilize specific combinations of inert and reactive gases, contingent on the exact nature of the composition containing ultra high molecular weight polyethylene to be processed. The gases should be capable of creating a plasma. The plasma, when appropriately generated and typically in conjunction with a dynamic masking process, may etch surfaces of the monofilament materials.

In embodiments, radio frequency (RF) generated cold plasmas in the presence of inert gases and/or reactive gases sustained in a reaction chamber may be used to modify and micro-sculpt surfaces of monofilaments. The surface modification effects may be achieved through the dry, chemical etching action of plasma particles. Etching occurs through chemical reactions between reactive plasma species and the monofilament surface to produce reaction products which may be removed from the system either as volatile reaction products or complexed with other agents (e.g., water vapor).

One embodiment for surface etching uses a generated plasma, housed in a chamber capable of sustaining the plasma at low pressures and with the capability to vary the plasma gas flow rates. In general, the method utilizes a low power-to-surface-area radio frequency generated plasma operated at relatively low vacuums, as compared to the high power levels and ultra-low pressures commonly used in the semiconductor industry.

The plasma method frequently employs the use of contaminant or extrinsic species that may or may not be reactive with the monofilament but which may, in some way, promote an integrated interaction with the monofilament and the plasma. These extrinsic species may originate from the reaction chamber wall residues, the weak vacuum conditions, and residual atmospheric substances. Plasma contaminants may include water vapor, carbon dioxide, dust/particulates, and/or sputter ions from holder materials, chamber walls or specific sputtering targets. The presence of extrinsic species during the etching process can result in irregular etching. The irregular etching may be due to random local fluctuations in the plasma field or to variable random masking of the monofilament surface from the applied plasma by the extrinsic species.

Further, in some embodiments the plasma etching process may be characterized by a dynamic masking, promoted by the presence of extrinsic species (e.g., water vapor, carbon dioxide, hydrocarbons, particulates, etc.), that are expected to be found in the reactor chamber environment at the relatively low pressures used (e.g., less than about 10 Torr).

The intensity and quality of the plasma to which a monofilament material is exposed can be varied over time and space, producing a randomized, irregularly etched surface that may be characterized by dimensional (i.e., depth and width) and morphological (i.e., geometry and porosity density) variations on the monofilament surface, having relief depths from at least about 1µm to about 20 µm, surface cavity diameters from about 1 µm to about 3 µm, and porosity densities from about 4 to about 120 pores per µm². In order to establish this plasma, low background pressures and relatively low power-to-surface-area levels may be employed.

As noted above, in some embodiments radio frequency (RF) generated plasma may be used. However, cold plasmas may also be generated by alternative methods (e.g., microwaves or direct current). Low pressure cold plasmas can be generated with radio frequencies of from about 10 kHz to about 27 MHz; at pressures from about 0.01 to about 0.20 Torr; with gas flows ranging from about 10 to about 200 standard cubic centimeters per min (sccm); with gas temperatures from about 300° Kelvin to about 600° Kelvin; with ion energies (potential) from about 10 to about 500 electron volts (eV); and approximate RF power densities from about 0.05 to about 1 watts (W) per cm².

In embodiments, a noble gas may be used to cool and stabilize the plasma and a reactive gas may be used to effect the actual chemical etching process. In embodiments, argon or helium are typical inert, noble gases. Reactive gases may be used to create chemical species that will react with the target surface; the type of reactive gas may be dependent on the material to be etched. The appropriate selection of the reactive gas requires that volatile reaction products be created in the reaction between the reactive gas plasma species and the target material, creating species that may be redeposited onto the surface and/or be carried away from the surface via the reaction chamber vacuum system. Useful reactive gases used to etch a material may be selected from the repertoire of those well versed in the art of cold plasma, dry etching processes. In particular, those combinations of plasma gases that have been used in the semiconductor industry for regular etching and/or cleaning of circuit boards and electronic components may be suitable. Suitable guidance can be found in the Handbook of Plasma Processing Technology (Rossnagel, et al. (editors); Noyes Publications, Westwood, New Jersey; 1990) and Cold Plasmas in Materials Fabrication (Grill, Alfred; IEEE Press, Piscataway, New Jersey; 1993), herein incorporated by reference. Generally, candidate volatile reaction products of the specific material surface will be identified, and reactive gases used in the RF plasma will be selected based on their potential to form the volatile species.

Noble gases may act to stabilize, cool and dilute the reactive gases in the plasma, while halogen-based gases create the chemically reactive etching species. In some embodiments, inert gases, such as argon or helium, act as carrier gases when bubbled through liquid reactants, thereby increasing the evaporation rate of the liquid reactant into its vapor state.

In embodiments, water vapor can constitute the reactive gas and argon the plasma stabilizing/cooling gas. Water molecules may be dissociated in the plasma to form activated moieties, such as OH⁻ and H⁺ radicals and/or other charged species. These species break polymer bonds and/or react with the organic components of the polymeric surface to create a more roughened and porous morphology.

In some embodiments, conditions for etching the surface of the present monofilaments employ either a He plasma, a F₂/He plasma, or a CF₄/He plasma with an operating temperature from about 25° C to about 100° C and pressure from about 0.005 to about 0.20 Torr. RF power levels should be from about 10 to about 200 watts.

In those cases where suitable matches between plasma gases and the monofilament are not known, the use of etching feasibility studies may be required. Testing should be conducted in such a manner as to be able to determine the volatility of the plasma reaction products, their redeposition characteristics, the type of surface morphology changes, and the parameters of the plasma system used to bring about the etching reaction.

The following steps are suggested when performing feasibility tests: (1) chemically identifying the material to be etched; (2) determining the vapor pressures of candidate volatile reaction products capable of being generated from the material being etched; (3) devising reaction scenarios that will generate the volatile reaction products; (4) identifying reactive plasma gases capable of producing these reaction products by interactions with the target material; and (5) establishing a reactive etching system, using the selected reactive plasma gases in combination with a suitable reaction chamber, appropriate radio frequencies and RF power levels, any other power levels, vacuum levels, flow rates and concentrations, and etching times.

In addition to the reactive and inert gases used in the plasma, in some embodiments hydrogen gas may be added to remove oxygen atoms from a monofilament surface, or to retard the etch rate. In still other embodiments, oxygen may be introduced into the plasma to accelerate etch rates. In some embodiments, O₂ may be used to oxidize harmful etching reaction by-products into a volatile species or also can be used to remove unwanted residual organic species by the technique known as plasma ashing.

Additional surface treatment processes may be applied to monofilaments previously conditioned by plasma etching. These optional, post-etch procedures may include, but are not limited to, some, or a combination, of the following commonly used surface coating/treatment methods: wet or dry coating applications, plating, vapor deposition, anodizing, surface polymerization, and re-etching of previously coated and/or etched surfaces of a device. Post-etch operations can be employed to either prepare the surface for a desired morphology or to make the surface smooth without any configuration.

Immobilized artificial coatings can be applied to monofilaments to enhance biocompatibility. Plasma pre-treatment may enhance adherence of a coating to monofilament surfaces by improving the adhesion characteristics of the monofilament, which in turn provides for better coating uniformity and thickness of biocompatible polymeric materials because the plasma treatment roughens and changes the micro-morphological configurations of the surface. Some immobilized polymeric coatings that can be used include: polyolefins, polyamides, polyimides, polyethers, polyesters, polystyrenes, polyvinyl chlorides, polypropylenes, polyisoprenes, polytetrafluoroethylenes, polyurethanes, polycarbonates, polyalkylimines, optionally in combination with cross-linking agents such as glutaraldehyde, glyoxal, malonaldehyde, succinaldehyde, adipaldehyde, or dialdehyde starch. U.S. Patent No. 5,415,938, the entire disclosure of which is incorporated by reference herein, identifies some of the existing art used to polymer coat medical implant devices. Chemical modifications can include surface polymerization via plasma reactions; polymer application via sprays, dipping, or cold vapor deposition; acid etching; electroplating; and/or passivation.

In another aspect, the present disclosure embraces a method for improving the handling characteristics of a monofilament suture by utilizing a plasma polymerization process to apply to the suture a coating including a siloxane polymer. Coatings may be formed by a plasma polymerization process whereby aliphatic hydrocyclosiloxane monomers are polymerized on the surface of the suture to form a siloxane coating on the suture. In one embodiment, amine groups may be introduced onto the polymer coating by co-polymerizing an organo-based monomer with the aliphatic hydrocyclosiloxane monomer or by carrying out a second plasma polymerization process for the introduction of the organo-based monomer. The amine groups on the polymer coating may then be reacted with carbonate polyoxyalkylenes to give polyoxyalkylene modified polymer coatings which enhance the handling characteristics of the coated sutures. Such a process is described in detail in WO03/037156A2, the disclosure of which is incorporated herein in its entirety by this reference.

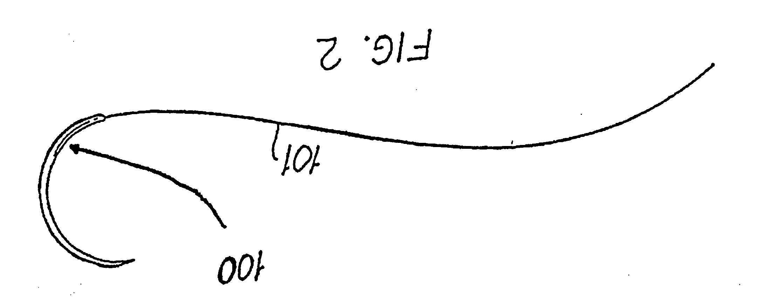

Sutures as described herein can be used to secure tissue in a desired position. As shown in FIG. 2, suture 101 may be attached to a surgical needle 100 by methods within the purview of those skilled in the art. Wounds may be sutured by approximating tissue and passing the needled suture through tissue to create wound closure. The needle is then typically removed from the suture and the suture tied.

The monofilaments prepared in accordance with this disclosure can have needles attached thereto using conventional techniques. In addition, the sutures can be packaged and sterilized using methods and materials within the purview of those skilled in the art.

While the above description contains many specifics, these specifics should not be construed as limitations on the scope of the invention, but merely as exemplifications of embodiments thereof. Those skilled in the art will envision many other possibilities within the scope and spirit of the invention as defined by the claims appended hereto.

## Claims

1. A suture comprising a monofilament comprising an ultra high molecular weight polyethylene possessing a weight average molecular weight of greater than about 400,000.

2. The suture of claim 1, wherein the monofilament further comprises at least one other component selected from the group consisting of polyvinyl ethers, poly(phenylene ethers), acetal resins, thermoplastic polyesters, polycarbonates, cellulose esters, thermoplastic polyamides, polyetheramides, polyesteramides, thermoplastic polyurethanes, polyvinylhalides, polyvinylidene halides, halogenated polyolefins, acrylic resins, polystyrenes, polyacrylonitriles, polyvinylesters, polyimides, polyetherimides, polysulfones, and combinations thereof.

3. The suture of claim 1, wherein the ultra high molecular weight polyethylene is blended with one or more materials selected from the group consisting of polyethylenes, polypropylenes, and copolymers of polyethylene and polypropylene.

4. The suture of claim 3, wherein the polypropylene is selected from the group consisting of isotactic polypropylene, atactic polypropylene, and syndiotactic polypropylene.

5. The suture of claim 3, wherein the blend of comprises from about 5 to about 95 weight percent ultra high molecular weight polyethylene.

6. The suture of claim 1, wherein the composition comprises a copolymer of an ultra high molecular weight polyethylene with one or more monomers selected from the group consisting of isotactic polypropylene, syndiotactic polypropylene and atactic polypropylene.

7. The suture of claim 1, wherein the ultra high molecular weight polyethylene possesses a weight average molecular weight of greater than about 1,000,000.

8. The suture of claim 1, wherein the ultra high molecular weight polyethylene resin possesses a melting point of from about 144° C to about 152° C.

9. The suture of claim 1, wherein the composition further comprises a fatty acid diester.

10. The suture of claim 9, wherein the fatty acid diester comprises a diester of a polyalkylene glycol.

11. The suture of claim 9, wherein the fatty acid diester comprises a polyalkylene glycol and a fatty acid selected from the group consisting of stearic acid, lauric acid, palmitic acid, myristic acid, arachidic acid, and behenic acid.

12. The suture of claim 1, further comprising a dye selected from the group consisting of carbon black, bone black, D&C Green No. 6, and D&C Violet No. 2.

13. The suture of claim 1, further comprising a roughened surface.

14. The suture of claim 1, further comprising a coating on at least a portion of the surface of the suture.

15. The suture of claim 14, wherein the coating is selected from the group consisting of polyolefins, polyamides, polyimides, polyethers, polyesters, polystyrenes, polyvinyl chlorides, polypropylenes, polyisoprenes, polytetrafluoroethylenes, polyurethanes, polycarbonates, and polyalkylimines optionally in combination with cross-linking agents selected from the group consisting of glutaraldehyde, glyoxal, malonaldehyde, succinaldehyde, adipaldehyde, and dialdehyde starch.

16. The suture of claim 14, wherein the coating comprises a siloxane polymer.

17. The suture of claim 16, wherein the coating comprises the product of plasma polymerization of aliphatic hydrocyclosiloxane monomers.

18. The suture of claim 17, wherein the coating further comprises amine groups.

19. The suture of claim 18, wherein the coating further comprises polyoxyalkylenes.

20. A surgical needle-suture combination comprising:
a surgical needle; and
a suture attached to the needle, the suture comprising a monofilament comprising an ultra high molecular weight polyethylene possessing a weight average molecular weight of greater than about 400,000.

21. A method of suturing a wound comprising:
providing a monofilament suture comprising an ultra high molecular weight polyethylene possessing a weight average molecular weight of greater than about 400,000 attached to a needle to form a needled suture; and
passing said needled suture through tissue to create wound closure.
